# EUROPÄISCHE PATENTSCHRIFT

(11) **EP 2 173 294 B1**
(45) Veröffentlichungstag und Bekanntmachung des Hinweises auf die Patenterteilung: **25.11.2020**
(21) Anmeldenummer: 08784746.3
(22) Anmeldetag: 12.07.2008
(51) Int. Cl.: A61F 13/00, A61F 13/36

(54) **MEDIZINISCHE KOMPRESSE II**
MEDICAL COMPRESS II
COMPRESSE MÉDICALE

(30) Priorität: 01.08.2007 DE 102007036082
(43) Veröffentlichungstag der Anmeldung: 14.04.2010
(73) Patentinhaber: Paul Hartmann AG, 89522 Heidenheim (DE)
(72) Erfinder: HOFSTETTER, Jürgen, 89522 Heidenheim (DE); SCHMID, Horst, 89567 Sontheim (DE); HALBAUER, Rainer, 73569 Obergröningen (DE)
(86) Internationale Anmeldenummer: PCT/EP2008/005717
(87) Internationale Veröffentlichungsnummer: WO 2009/015757

(56) Entgegenhaltungen:
- BE-A- 505 633
- GB-A- 782 861
- GB-A- 1 410 810

## Beschreibung

Die vorliegende Erfindung betrifft medizinische Kompressen aus einem textilen Flachbahnmaterial, insbesondere Mullkompressen, in einer anwenderfreundlichen Form. Weiterhin betrifft die Erfindung ein Verfahren zur Herstellung dieser Kompressen.

Medizinische Kompressen sind seit langer Zeit zur Behandlung von akuten Wunden in der Notfallmedizin oder zur Verwendung bei chirurgischen Eingriffen bekannt. Im Wesentlichen werden diese Kompressen hinsichtlich der eingesetzten Materialien unterschieden und mit Hilfe dieser Unterscheidung in Mullkompressen und Vliesstoffkompressen eingeteilt. Dabei werden Mullkompressen üblicherweise aus einem Baumwollgewebe hergestellt, das je nach Fadendichte eine grobe oder eine feine Gitterstruktur aufweist. Die Anforderungen an den Mull zur Bildung von Kompressen sind mit der DIN EN 14079 festgelegt.

Da es sich bei den Mullkompressen um eine Gitterstruktur handelt, die den Nachteil aufweist, dass endständige Fäden sich lösen können, sind vielfach Lösungen unterbreitet worden, dieses Ablösen zu verhindern. So wird beispielsweise mit der DE 2261889 eine Kompresse beschrieben, die mindestens einen vermaschten Streifen aufweist. Durch die Vermaschung der Fäden werden zwei gegenüberliegende Ränder eines Bandmaterials gebildet, aus denen keine Fäden ausgelöst werden können. Die Kompresse wird letztendlich aus einem Teilabschnitt dieses Bandmaterials gebildet, wobei die Schnittkanten dieses Abschnittes eingeschlagen werden. Darüber hinaus wird mit der DE 9014500 eine Mullkompresse vorgeschlagen, die in unmittelbarer Nähe zu einer Schnittkante thermoplastische Fäden, Bänder, Streifen oder Vliesstreifen aufweist. Diese zusätzlichen Materialien werden mit dem Mull verschweißt, versiegelt oder verklebt. Als im Markt bekannte Mullkompressen haben sich beispielsweise ES-Kompressen etabliert. Diesen vorgeschlagenen Lösungen oder vorhandenen Produkten ist gemeinsam, dass sie als zu aufwendig und/oder zu kostenintensiv in der Fertigung angesehen werden.

GB1410810 offenbart eine medizinische Kompresse umfassend mindestens acht Lagen eines textilen Flachbahnmaterials sowie ein Verfahren zur Herstellung einer derartigen Kompresse. Sämtliche Ausführungsformen weisen mindestens eine Randkante auf, welche eine Schnittkante darstellt. Eine derartige Kompresse birgt das Risiko des Einbringens von einer Schnittkante ausgefranster Fäden in eine zu behandelnde Wunde.

Aufgabe der vorliegenden Erfindung ist es, alternative, textile, medizinische Kompressen bereitzustellen, die einerseits in der Verwendung ein hohes Maß an Anwendungssicherheit bieten, und zugleich kostengünstig in der Fertigung sind. Des Weiteren soll eine Mehrzahl solcher Kompressen leicht stapelbar sein, wobei die Kompressen ein möglichst geringes Packvolumen einnehmen sollen. Zudem soll ein Verfahren zur Herstellung dieser Kompressen bereitgestellt werden.

Gelöst werden diese Aufgaben von einer medizinischen Kompresse gemäß Anspruch 1. Demnach umfasst eine erfindungsgemäße medizinische Kompresse mindestens 8 Lagen eines textilen Flachbahnmaterials, wobei jede Lage über mindestens eine Faltkante mit einer weiteren Lage verbunden ist, und mindestens zwei Faltkanten senkrecht zueinander stehen. Die Kompresse ist aus einem rechteckigen Materialabschnitt des Flachbahnmaterials mit zwei sich gegenüber liegenden parallelen Schnittkanten A und zwei sich gegenüber liegenden parallelen Schnittkanten B gebildet, wobei die Kantenlänge a der Schnittkanten A größer ist als die Kantenlänge b der Schnittkanten B. Die Kompresse ist derart gefaltet, dass die Kompresse eine aus mindestens einer ersten Faltkante und einer zweiten Faltkante gebildete erste Einfasskante umfasst, wobei die erste Faltkante direkt benachbarte Lagen oder Teilabschnitte der direkt benachbarten Lagen verbindet und parallel zu den Schnittkanten B gebildet ist, und die zweite Faltkante die äußeren, die Auflageflächen der Kompresse bildenden Lagen verbindet. Dabei ist vorgesehen, dass jede Randkante der Kompresse ausschließlich aus Faltkanten und/ oder Einfasskanten gebildet ist. Dabei handelt es sich um eine Kompresse, die im Querschnitt betrachtet mindestens einen ersten 8-lagigen Bereich und einen zweiten 12-lagigen Bereich aufweist. Die Kompresse ist derart gefaltet, dass die äußeren, die Auflageflächen der Kompresse bildenden Lagen die Form eines Rechtecks oder eines Quadrates aufweisen. Bevorzugt ist dabei vorgesehen, dass die erste Einfasskante mindestens zwei, insbesondere vier direkt benachbarte Lagen verbindende erste Faltkanten umfasst.

Im Zusammenhang mit der vorliegenden Erfindung soll unter einer Faltkante eine Kante oder ein Teilabschnitt der Kante verstanden sein, die durch das vollständige oder teilweise Übereinanderlegen, Übereinanderschlagen oder Einschlagen von zwei Untereinheiten des Materialabschnitts gebildet wird, wobei die beiden Untereinheiten des Materialabschnitts mittels der Faltkante verbunden sind. Hierbei können die beiden Untereinheiten des Materialabschnitts nach dem Übereinanderlegen, Übereinanderschlagen oder Einschlagen direkt benachbart sein, das heißt, dass die beiden Untereinheiten des Materialabschnitts im direkten Kontakt stehen, oder durch weitere Lagen beabstandet sein. Sind die übereinander gelegten oder geschlagenen Teilabschnitte durch mindestens eine weitere Lage beabstandet, wird im Zusammenhang mit der vorliegenden Erfindung von einer Einfasskante gesprochen. Die Einfasskante wird hierbei aus mindestens einer Schnittkante und/ oder mindestens einer Faltkante und der von dieser Schnitt- oder Faltkante verschiedenen, die übereinander gelegten oder geschlagenen Teilabschnitte verbindenden Faltkante gebildet, wobei die beteiligten Kanten direkt an- oder gegeneinander liegen. Die außen liegende, die beiden übereinander gelegten oder geschlagenen Teilabschnitte verbindenden Faltkanten umschließen damit die innen liegenden Schnittkanten und/ oder Faltkanten, wobei die Einfasskante aus der Schnitt- und/ oder Faltkante und der die übereinander gelegten oder geschlagenen Teilabschnitte verbindenden Faltkante gebildet ist. Idealer Weise liegen hierbei die außen liegende Faltkante und die innen liegenden Schnitt-, und/ oder Faltkanten direkt an- oder gegeneinander. In diesem Fall weisen die Kanten keinen Abstand auf. Es kann jedoch auch vorgesehen sein, dass die Schnitt- und/ oder Faltkanten von der übereinander gelegten oder geschlagenen Teilabschnitte verbindenden Faltkante durch weitere Einfasskanten beabstandet sind. Damit werden gemäß der vorliegenden Erfindung auch solche Kanten als Einfasskanten bezeichnet, deren beteiligte Schnitt- und/ oder Faltkanten geringfügig, das heißt höchstens 10 %, von der außen liegende Faltkante beabstandet sind. Hierbei soll der jeweilige Wert des Abstands auf die Länge einer Randkante der Kompresse im endgefalteten Zustand bezogen werden, wobei als Bemessungsgrundlage diejenige Randkante der Kompresse verwendet wird, die dem Betrag nach die größte Kantenlänge aufweist.

Eine Schnittkante eines Materialabschnitts des Flachbahnmaterials ist demgegenüber eine Kante, die durch das Trennen von einem ersten Materialabschnitt aus einem den ersten Materialabschnitt umfassenden größeren Materialabschnitt gebildet wird, wobei jeweils eine Schnittkante dem ersten Materialabschnitt und eine Schnittkante dem verbleibenden Materialabschnitt zugeordnet werden kann. Hierbei können alle heute bekannten Trenntechniken, wie das Schneiden mittels Messer oder Scheren, Laserstrahlen, Wasserstrahlen und andere Techniken eingesetzt werden.

Weiterhin soll unter einer Stoßkante eine Schnitt- oder Faltkante sowie Teilabschnitte dieser Schnitt- oder Faltkanten verstanden sein, die durch das Anlegen dieser Schnitt- oder Faltkante oder dieser Teilabschnitte der Schnitt- oder Faltkanten gegen eine weitere Schnittkante oder eine weitere Faltkante bzw. Teilabschnitte der weiteren Schnittkante oder weiteren Faltkante gebildet wird, wobei die beteiligten Schnitt- oder Faltkanten bzw. deren Teilabschnitte in einer Ebene liegen. Idealer Weise liegen die Stoßkanten einer erfindungsgemäßen Kompresse direkt an- oder gegeneinander. In diesem Fall weisen die Kanten keinen Abstand auf. Gemäß der vorliegenden Erfindung werden auch solche Kanten als Stoßkanten bezeichnet, deren beteiligte Schnitt- oder Faltkanten geringfügig, das heißt höchstens 15 %, von einander beabstandet sind und/ oder bis zu 15 % überlappen, wobei der jeweilige Wert des Abstandes oder der Überlappung auf die Länge einer Randkante der Kompresse im endgefalteten Zustand bezogen werden, wobei als Bemessungsgrundlage diejenige Randkante der Kompresse verwendet wird, die dem Betrag nach die größte Kantenlänge aufweist.

Des Weiteren soll im Zusammenhang mit der vorliegenden Erfindung (falls nicht anders angezeigt) unter einer Randkante eine Außenkante der endgefertigten Kompresse verstanden sein.

Durch die Ausbildung von einer erfindungsgemäßen Einfasskante, die eine Randkante der endgefertigten Kompresse bildet, kann eine Kompresse bereit gestellt werden, die keine freien Schnittkanten aufweist und zudem eine Materialersparnis und damit eine Kostenreduktion in der Fertigung ermöglicht. Durch die Ausbildung einer Einfasskante aus Faltkanten wird verhindert, dass sich Fäden vom Schnittrand lösen und bei der bestimmungsgemäßen Verwendung in eine Wunde gelangen. Zudem weist eine erfindungsgemäße Kompresse den Vorteil auf, dass auch beim einmaligen Entfalten der fertigen insbesondere mindestens 8-lagigen Kompresse keine Schnittkanten freigelegt werden. Diese Kompresse ist besonders handhabungssicher und darüber hinaus anwenderfreundlich, da der Anwender selbst festlegen kann, ob er beispielsweise eine
8-lagige Kompresse als eine 4-lagige oder als eine 8-lagige Kompresse nutzen möchte. In beiden Fällen liegen keine Schnittkanten frei, d.h., dass jede Schnittkante des Materialabschnitts von mindestens einer Materiallage abgedeckt ist.

Grundsätzlich kann eine Kompresse aus einem rechteckigen Materialabschnitt eines Flachbahnmaterials mit zwei sich gegenüber liegenden parallelen Schnittkanten A und zwei sich gegenüber liegenden parallelen Schnittkanten B gefertigt werden, wobei die Kantenlänge a der Schnittkanten A größer ist als die Kantenlänge b der Schnittkanten B. Bei der Ausbildung einer Kompresse mit einer erfindungsgemäßen Einfasskante, die mindestens eine erste Faltkante, die parallel zu den kleineren Schnittkanten B gebildet wird, und eine Faltkante, die diese erste Faltkante umschließt, umfasst, kann im Vergleich zu einer Kompresse mit einer Einfasskante, die eine erste Faltkante, die parallel zu den größeren Schnittkanten A gebildet wird, und eine Einfasskante umfasst, die diese erste Faltkante umschließt, eine erhebliche Materialersparnis verwirklicht werden. Diese Materialersparnis beläuft sich z.B. für eine quadratische, 8-lagige Kompresse bei gleichem Material in Abhängigkeit von der Auflagefläche und der Breite der zuerst umgeschlagenen Teilbereiche auf 5 - 15 %.

Als vorteilhaft hat sich weiterhin herausgestellt, wenn die Kompresse derart gefaltet ist, dass die äußeren, die Auflageflächen der Kompresse bildenden Lagen die Form eines Rechtecks oder eines Quadrates aufweisen. Insbesondere ist die medizinische Kompresse derart gefaltet, dass die äußeren, die Auflageflächen der Kompresse bildenden Lagen vollständig durch jeweils einen zusammenhängenden Bereich des Flachbahnmaterials gebildet sind. Diese die Auflageflächen bildenden Lagen weisen also keine Stoßkanten auf. Insbesondere ist die medizinische Kompresse derart gefaltet, dass die äußeren, die Auflageflächen der Kompresse bildenden Lagen vollständig durch jeweils einen zusammenhängenden Bereich des Flachbahnmaterials gebildet sind, wobei die Auflageflächen rechteckig oder quadratisch sind.

Diese Auflageflächen können verwirklicht werden, indem eine erfindungsgemäße Kompresse aus einem rechteckigen Materialabschnitt des Flachbahnmaterials mit zwei sich gegenüber liegenden parallelen Schnittkanten A und zwei sich gegenüber liegenden parallelen Schnittkanten B gefertigt wird, wobei die Kantenlänge a der Schnittkanten A größer als die Kantenlänge b der Schnittkanten B ist, und jede Schnitt-, Falt-, Stoß-, Einfass- und/ oder Randkante der Kompresse parallel oder senkrecht zu einer weiteren Schnitt-, Falt-, Stoß-, Einfass- und/ oder Randkante der Kompresse gebildet ist und wobei mindestens eine Schnitt-, Falt-, Einfass- oder Stoßkante der Kompresse senkrecht zu einer weiteren Schnitt-, Falt-, Einfass- oder Stoßkante der Kompresse gebildet ist. Weiterhin bevorzugt bilden hierbei diejenigen Faltkanten, die parallel zu den Schnittkanten B des Materialabschnitts gebildet sind, die ersten Faltkanten. Weiterhin bevorzugt bilden hierbei diejenigen Faltkanten, die parallel zu den Schnittkanten B des Materialabschnitts gebildet sind, die Faltkanten, die zusammen mit einer weiteren Faltkante der Kompresse die erste Einfasskante der endgefertigten Kompresse bilden, wobei ganz besonders bevorzugt die Schnittkanten A erste Stoßkanten der Kompresse bilden. Diese zu den Schnittkanten B parallel gebildeten Faltkanten verbinden zwei direkt benachbarte Lagen der Kompresse miteinander. Dagegen verbindet die weitere Faltkante die beiden die Auflageflächen der Kompresse bildenden Lagen miteinander. Ganz besonders bevorzugt bilden diejenigen Faltkanten, die parallel zu den Schnittkanten B des Materialabschnitts gebildet sind, die ersten Faltkanten, wobei diese ersten Faltkanten zusammen mit der weiteren Faltkante die erste Einfasskante der endgefertigten Kompresse bilden, wobei ganz besonders bevorzugt die Schnittkanten A erste Stoßkanten der Kompresse bilden. Dabei kann weiterhin vorzugsweise vorgesehen sein, dass Teilabschnitte der Schnittkanten B in Aufsicht der Kompresse betrachtet innerhalb der Kompresse deckungsgleich angeordnet liegen.

Damit weist eine erfindungsgemäße Kompresse vorzugsweise auch rechteckige oder quadratische Auflageflächen mit den Randkanten C und D auf, wobei die Kantenlänge c der Randkanten C größer oder gleich der Kantenlänge d der Randkanten D ist, und ist vorzugsweise derart gefaltet, dass Teilabschnitte der Schnittkanten B der Kompresse in Aufsicht deckungsgleich angeordnet sind und insbesondere parallel zu den Randkanten D liegen. Insbesondere weist eine erfindungsgemäße Kompresse quadratische Auflageflächen F mit Randkanten D mit der Kantenlänge d auf. Es kann jedoch auch vorgesehen sein, dass eine erfindungsgemäße Kompresse rechteckige Auflageflächen mit den Randkanten C und D aufweist, wobei die Kantenlänge c der Randkante C größer ist als die Kantenlänge d der Randkante D, und parallel zu der Randkante D liegende erste Faltkanten und weitere Faltkanten als Einfasskante aufweist. In diesem Fall liegen die Schnittkanten A des rechteckigen Materialabschnitts parallel zu den Randkanten C und die Schnittkanten B des rechteckigen Materialabschnitts parallel zu den Randkanten D. Anzumerken ist an dieser Stelle, dass erfindungsgemäße Kompressen sowohl maschinell als auch von Hand gefertigt werden können.

Erfindungsgemäß weist eine anspruchsgemäße Kompresse mindestens 8 und höchstens 12 Lagen auf. Dabei handelt es sich bei einer erfindungsgemäßen Kompresse um eine Kompresse, die im Querschnitt betrachtet mindestens einen ersten 8-lagigen Bereich und einen zweiten 12-lagigen Bereich aufweist. Hierunter ist zu verstehen, dass die Kompresse im Querschnitt betrachtet nicht über den gesamten Bereich ihrer Quer- oder Längserstreckung einen homogenen Lagenaufbau aufweist, sondern dass die Kompresse in einem ersten Teilbereich 12-lagig und in mindestens einem weiteren Teilbereich 8-lagig ist. Insbesondere weist die Kompresse einen ersten 8-lagigen Randbereich und einen zweiten 12-lagigen Randbereich auf.
Erfindungsgemäß ist vorgesehen, dass jede Randkante der Kompresse ausschließlich aus Faltkanten und/ oder Einfasskanten gebildet ist. Diese Kompressen weisen den Vorteil auf, dass sie besonders handhabungssicher sind. Diese Anordnung der Faltkanten weist weiterhin den Vorteil auf, dass eine minimale Eingriffswahrscheinlichkeit in die Kompresse besteht. Das heißt, selbst wenn die erfindungsgemäßen Kompressen in einem Stapel angeordnet sind, kann eine Kompresse ergriffen werden ohne unbeabsichtigt eine weitere Lage einer benachbarten Kompresse zu ergreifen. Dies ist insbesondere der Fall, wenn eine Kompresse beispielsweise Randkanten aufweist, die Schnittkanten des zugrunde liegenden Materialabschnitts enthalten. In einer weiteren Ausführung der Erfindung kann auch vorgesehen sein, dass die Kompresse zwei weitere Schnitt- und/ oder Faltkanten als Stoßkanten umfasst. Insbesondere liegen diese Stoßkanten parallel zu der zweiten Faltkante. Weiterhin bevorzugt liegen die Stoßkanten senkrecht zu der Einfasskante. Ganz besonders bevorzugt ist die Kompresse derart gefaltet, dass die Schnittkanten A oder Teilbereiche der Schnittkanten A als Stoßkanten ausgebildet sind. Somit kann sichergestellt werden, dass alle Schnittkanten im Inneren der Kompresse angeordnet werden. Somit kann insbesondere auch sichergestellt werden, dass alle Randkanten der Kompresse aus Faltkanten gebildet werden.

Gemäß einer weiteren Ausführung der vorliegenden Erfindung ist vorgesehen, dass die Schnittkanten B oder ein Teilabschnitt der Schnittkanten B in jedem Punkt einen Abstand zu der parallelen ersten Einfasskante von mindestens 15 % und höchstens 85 % des Betrags der Länge einer senkrecht zur ersten Einfasskante liegenden Randkante aufweist. Besonders bevorzugt weist die Kompresse Schnittkanten B oder einen Teilabschnitt hiervon auf, die in jedem Punkt einen Abstand zu einer parallelen ersten Einfasskante von mindestens 20 % und höchstens 60 % des Betrags der Länge einer senkrecht zur ersten Einfasskante liegenden Randkante aufweist. Ganz besonders bevorzugt weist diese Kompressen Schnittkanten B oder einen Teilabschnitt der Schnittkanten B auf, die in jedem Punkt einen Abstand zu einer parallelen ersten Einfasskante von mindestens 20 % und höchstens 40 % des Betrags der Länge einer senkrecht zur ersten Einfasskante liegenden Randkante aufweist.

Zur Herstellung einer erfindungsgemäßen Kompresse kann als Flachbahnmaterial jedwede textile Flachbahnmaterialien eingesetzt werden, die verschieden von nichtgewebten Flachbahnmaterialien, den so genannten Nonwoven oder Vliesstoffen sind. Die vorliegende Erfindung betrifft keine Vliesstoffkompressen. Als textile Flachbahnmaterialien können erfindungsgemäß insbesondere Gewebe, Gewirke oder Gestricke zum Einsatz gebracht werden. Ganz besonders bevorzugt werden Gewebe und insbesondere Gewebe mit einer Leinwandbindung zum Einsatz gebracht.

Die textilen Flachbahnmaterialien können weiterhin bevorzugt aus einem Garn- oder Fasermaterial hergestellt sein, das Fasern oder Filamente natürlichen Ursprungs und/ oder synthetische Fasern umfasst. Als Fasern natürlichen Ursprungs umfasst eine erfindungsgemäße Kompresse insbesondere Fasern aus Baumwolle, Hanf, Flachs oder Leinen. Falls das Flachbahnmaterial Garn- oder Fasermaterial umfasst, das synthetische Fasern umfasst, so können hierzu Fasern oder Filamente aus Viskose, Polyester, Celluloseacetat, Carboxymethylcellulose, Hydroxyethylcellulose verwendet werden. Ganz besonders bevorzugt umfasst das textile Flachbahnmaterial ein Garn- oder Fasermaterial aus Baumwolle und/ oder Viskose, das den Anforderungen der DIN EN 14079 genügt. Diese Materialien, insbesondere Mull, sind im Vergleich zu bekannten nicht-textilen oder nicht-gewebten Flachbahnmaterialien wie Nonwoven oder Vliesstoffe nicht in einem stufenlosen, kontinuierlichen Verfahren zu verarbeiten. Aus diesem Grund müssen erfindungsgemäße Kompressen aus einem diskreten Materialabschnitt gefertigt werden.

Eine besonders bevorzugte Ausführung einer erfindungsgemäßen medizinischen Kompresse ist eine Mullkompresse. Diese Mullkompresse umfasst mindestens 8 Lagen Mull gemäß DIN EN 14079, wobei jede Lage über mindestens eine Faltkante mit einer weiteren Lage verbunden ist und wobei mindestens eine erste Faltkante und eine zweite Faltkante senkrecht zueinander stehen. Die Mullkompresse ist derart gefaltet, dass die Kompresse mindestens zwei Faltkanten als erste Stoßkanten umfasst, wobei jede dieser ersten Stoßkanten direkt benachbarte Lagen verbindet. Insbesondere weist die medizinische Kompresse zwei Faltkanten als erste Stoßkanten und zwei Schnittkanten als zweite Stoßkanten auf. Des Weiteren kann diese Mullkompresse alle weiteren Merkmale der zuvor beschriebenen Art einzeln oder in Kombination umfassen.

Somit wird mit der vorliegenden Erfindung eine Kompresse bereitgestellt, die derart gefaltet ist, dass die eingeschlagenen Schnittkanten wie bei bekannten ES-Kompressen am Rand übereinander liegen. Im Gegensatz zu diesen ES-Kompressen weisen erfindungsgemäße Kompressen jedoch in diesem Randbereich lediglich 12 Lagen und nicht wie die ES-Kompresse 16 Lagen auf. Somit wird eine Kompresse bereitgestellt, die flacher ist und damit weniger Lagerraum einnimmt. Durch die erfindungsgemäße Faltung kann nun auch eine Kompresse bereitgestellt werden, die viel leichter stapelbar ist.

Gemäß einem weiterführenden Gedanken der vorliegenden Erfindung ist auch ein Kompressenstapel umfassend eine Mehrzahl von erfindungsgemäßen medizinischen Kompressen Gegenstand der vorliegenden Erfindung. Dieser Stapel umfasst eine Mehrzahl an Kompressen der zuvor beschriebenen Art. Insbesondere kann dieser Stapel eine Mehrzahl an gleichen Kompressen umfassen, wobei jede Kompresse einzelne Merkmale oder Kombinationen von Merkmalen der zuvor beschriebenen Kompressen aufweist.

Durch die Anordnung von Faltkanten als erste Einfasskante kann ein Kompressenstapel bereitgestellt werden, der im Vergleich zu im Markt befindlichen Kompressen in sich stabiler ist und weniger Platz einnimmt. Hierdurch kann insbesondere Verpackungsmaterial eingespart werden. Werden beispielsweise im Markt befindliche ES-Kompressen gestapelt, so weist die Verpackung mit einem Kompressenstapel von 100 Kompressen eine Höhe von 155 mm (Außenmaß der Verpackung) auf. Werden dagegen erfindungsgemäße Kompressen gestapelt (100 Stück) unter denselben Bedingungen verpackt, so ist ein Außenmaß von 139 mm zu verzeichnen (dieselben Messbedingungen). Somit kann insbesondere Verpackungsmaterial und Lagerraum eingespart werden.

Weiterhin bevorzugt weist ein erfindungsgemäßer Stapel Kompressen mit rechteckigen oder quadratischen Auflageflächen auf, wobei jede Kompresse zwei sich gegenüberliegende Randkanten C mit einer Kantenlänge c und zwei sich gegenüberliegende Randkanten D mit einer Kantenlänge d aufweist, und wobei die Kantenlänge c größer oder gleich der Kantenlänge d ist. Insbesondere weist dieser Stapel eine Mehrzahl an Kompressen mit quadratischen Auflageflächen auf.

In einer weiteren Ausführung der vorliegenden Erfindung weist ein erfindungsgemäßer Kompressenstapel eine Mehrzahl an Kompressen auf, die aus einem rechteckigen Materialabschnitt eines textilen Flachbahnmaterials mit zwei sich gegenüber liegenden parallelen Schnittkanten A und zwei sich gegenüber liegenden parallelen Schnittkanten B gebildet sind, wobei die Kantenlänge a der Schnittkanten A größer ist als die Kantenlänge b der Schnittkanten B. Ganz besonders bevorzugt weist ein erfindungsgemäßer Kompressenstapel eine Mehrzahl an Kompressen auf, deren Schnittkanten B oder Teilabschnitte der Schnittkanten B in jedem Punkt einen Abstand zu einer parallelen ersten Einfasskante von mindestens 15 % und höchstens 85 % des Betrags der Länge einer zweiten, senkrecht zur ersten Einfasskante liegenden Randkante aufweisen.

Diese Kompressen sind derart übereinander gestapelt, dass jeweils eine erste Auflagefläche einer ersten Kompresse deckungsgleich mit einer ersten Auflagefläche einer zweiten oder weiteren Kompresse aufeinander liegt. Hierbei kann weiterhin bevorzugt sein, dass jede erste Einfasskante einer Kompresse, die eine zu den Schnittkanten B gebildeten Faltkante und eine weitere Faltkante umfasst, die beiden äußeren, die Auflageflächen der Kompresse bildenden Lagen des textilen Flachbahnmaterials miteinander verbindet, deckungsgleich übereinander liegen.

Auch ein Verfahren zur Herstellung einer medizinischen Kompresse mit mindestens 8 Lagen eines textilen Flachbahnmaterials, wobei jede Lage über mindestens eine Faltkante mit einer weiteren Lage verbunden ist und mindestens zwei Faltkanten senkrecht zueinander stehen, ist beschrieben. Insbesondere soll ein Verfahren zur Herstellung einer Kompresse der zuvor beschriebenen Art beschrieben werden. Das Verfahren umfasst die Verfahrensschritte
a) Bereitstellen eines rechteckigen Materialabschnitts des Flachbahnmaterials mit zwei sich gegenüberliegenden ersten Schnittkanten A und zwei sich gegenüberliegenden zweiten Schnittkanten B, wobei die Kantenlänge a der Schnittkante A größer ist als die Kantenlänge b der Schnittkante B,
b) Einschlagen der Schnittkanten B des rechteckigen Materialabschnitts entlang einer ersten zu den Schnittkante B parallelen Faltlinien und einer zweiten zu den Schnittkante B parallelen Faltlinien zur Ausbildung von zwei ersten Faltkanten,
c) Einschlagen mindestens einer der unter b) ausgebildeten Faltkanten entlang einer dritten, zu einer Schnittkante B parallel liegenden Faltlinie zur Ausbildung einer weiteren Faltkante oder einer Einfasskante,
d) Einschlagen der unter c) ausgebildeten Faltkante oder Einfasskante entlang einer vierten, zu einer Schnittkante B parallel liegenden Faltlinie zur Ausbildung einer ersten Einfasskante, die mindestens eine erste Faltkante und eine zweite Faltkante umfasst und
e) weiteres Einschlagen von Schnittkanten oder gebildeten Falt- oder Einschlagkanten zur Ausbildung von Stoßkanten oder weiterer Falt- oder Einschlagkanten.

Insbesondere erfolgt innerhalb des Verfahrensschrittes e) ein Einschlagen der ersten Schnittkanten A des rechteckigen Materialabschnitts zur Ausbildung von zwei Stoßkanten.

Weiterhin beschrieben ist, dass innerhalb des Verfahrensschrittes b) ein Einschlagen der ersten Schnittkanten B des rechteckigen Materialabschnitts erfolgt, wobei jede Faltlinien in jedem Punkt mindestens 5 mm und höchstens 30 mm von der nächstliegenden Schnittkante B entfernt ist.

Mit einem derartigen Verfahren soll insbesondere ein Verfahren zur Herstellung einer medizinischen Kompresse bereitgestellt werden, die mindestens 8 und höchstens 12 Lagen eines Flachbahnmaterials umfasst. Ganz besonders bevorzugt handelt es sich bei einemderartigen Verfahren um ein Verfahren zur Herstellung einer Kompresse, die im Querschnitt betrachtet mindestens einen ersten 8-lagigen Bereich und einen zweiten 12-lagigen Bereich aufweist. Hierunter ist zu verstehen, dass die Kompresse im Querschnitt betrachtet nicht über den gesamten Bereich ihrer Quer- oder Längserstreckung einen homogenen Lagenaufbau aufweist, sondern dass die Kompresse in einem ersten Teilbereich 12-lagig und in mindestens einem weiteren Teilbereich 8-lagig ist. Insbesondere weist die Kompresse einen ersten 8-lagigen Randbereich und einen zweiten 12-lagigen Randbereich auf.
Damit ist gemäß einem derartigen Verfahren vorgesehen, dass innerhalb des Verfahrensschrittes c) ein Einschlagen beider im Verfahrensschritt b) gebildeten Faltkanten zur Ausbildung von zwei Einfasskanten erfolgt. Insbesondere erfolgt ein symmetrisches Einschlagen.

In einem alternativen Verfahren kann auch vorgesehen sein, dass innerhalb des Verfahrensschrittes c) ein insbesondere symmetrisches Einschlagen beider im Verfahrensschritt b) gebildeten Faltkanten zur Ausbildung von zwei Einfasskanten und zur Ausbildung von zwei Stoßkanten erfolgt.

In einem weiteren alternativen Verfahren kann auch vorgesehen sein, dass innerhalb des Verfahrensschrittes c) ein Einschlagen einer Faltkante entlang einer dritten, zu einer Schnittkante B parallel liegenden Faltlinie auf die andere Faltkante erfolgt.

Insbesondere soll ein Verfahren zur Herstellung einer Kompresse mit quadratischen Auflageflächen F mit dem Flächenmaß d² beschrieben werden, wobei d die Kantenlänge einer Randkante D der Kompresse ist. In diesem Verfahren wird bevorzugt als Ausgangsmaterial ein rechteckiger Materialabschnitt mit den Schnittkanten A und B verwendet, wobei dieser Materialabschnitt weiterhin bevorzugt eine Kantenlänge a mit
a = 4 d + 2 e der Schnittkanten A und eine Kantenlänge b mit b = d + 2 e' der Schnittkanten B aufweist, wobei d die Kantenlänge der endgefalteten Kompresse, e die Kantenlänge eines Teilabschnitts der Schnittkante A mit e ≤ ½ d und e' die Kantenlänge eines Teilabschnitts der Schnittkante B mit e' ≤ ½ d ist. Auf diese Weise kann ohne großen Verschnitt oder Abfall lediglich durch präzise Faltung eine mindestens 8-lagige Kompresse und höchstens 10-lagige oder 12-lagige Kompresse bereitgestellt werden, die eine besonders gleichmäßige Materialverteilung über die Auflagefläche verteilt vorsieht.

Durch das hier beschriebene Verfahren, wird ein Verfahren beschrieben, das eine kostengünstige Fertigung einer medizinischen Kompresse gewährleistet.

Hervorzuheben ist an dieser Stelle, dass die hier aufgeführten Merkmale der bevorzugten oder alternativen Ausgestaltungen der Erfindungen nicht auf die einzelnen Bevorzugungen oder Alternativen zu beschränken sind. Es ist vielmehr der Fall, dass die Kombination der Ausgestaltungen bzw. die Kombination der Einzelmerkmale der alternativen Formen ebenso zu einer erfindungsgemäßen Ausgestaltung zu zählen ist. Ebenso wenig soll die Erfindung durch die nachfolgende Beschreibung der Zeichnungen reduziert verstanden sein. In den Zeichnungen zeigen:
- Figur 1:: Ein Materialabschnitt zur Herstellung einer erfindungsgemäßen Kompresse in Aufsicht
- Figur 2a:: Erstes Zwischenprodukt zur Herstellung einer erfindungsgemäßen Kompresse in Aufsicht
- Figuren 2b, 2c, 2d:: Zwischenprodukt gemäß Figur 2a in verschiedenen Querschnitten
- Figur 3a:: Zweites Zwischenprodukt zur Herstellung einer erfindungsgemäßen Kompresse in Aufsicht
- Figuren 3b, 3c, 3d:: Zwischenprodukt gemäß Figur 3a in verschiedenen Querschnitten
- Figur 4a:: Eine erfindungsgemäße Kompresse in Aufsicht
- Figuren 4b, 4c, 4d, 4e:: Die Kompresse gemäß Figur 4a in verschiedenen Querschnitten

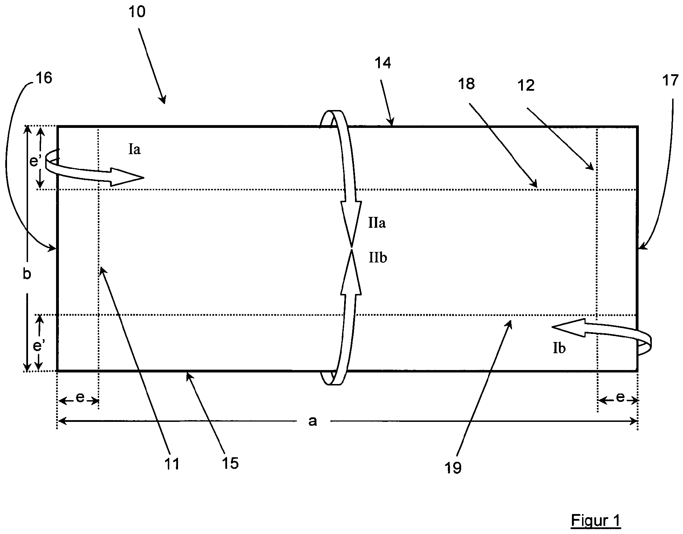

Mit Figur 1 ist ein rechteckiger Materialabschnitt (10) aus Mull gemäß DIN EN 14079 zur Herstellung einer erfindungsgemäßen Kompresse mit quadratischen Auflageflächen gezeigt. Dieser Materialabschnitt weist zwei sich gegenüberliegende erste Schnittkanten A (14, 15) mit der Kantenlänge a = 230,0 mm auf. Der Materialabschnitt weist weiterhin zwei sich gegenüberliegende zweite Schnittkanten B (16, 17) mit der Kantenlänge b = 99,0 mm auf.

Im Folgenden soll ein Verfahren zur Herstellung einer mindestens 8-lagigen Kompresse an Hand der Zeichnungen erläutert werden. Hierbei wird in einem ersten Verfahrensschritt der beschriebene rechteckige Materialabschnitt (10) bereitgestellt. In einem zweiten Verfahrensschritt werden die Schnittkanten B (16, 17) in Richtung der Pfeile Ia und Ib entlang der Faltlinien I (11, 12) zur Bildung von ersten Faltenkanten G (26) und G' (27) auf eine Oberseite des Materialabschnittes umgeschlagen. Durch das Umschlagen einer Schnittkante B (16, 17) werden zwei Untereinheiten des Materialabschnitts aufeinander gelegt, so dass die umgeschlagene Untereinheit als auch die verbleibende Untereinheit jeweils eine separate Lage bildet, wobei die gebildeten Lagen direkt benachbart und durch die gebildeten Faltkanten G (26) oder G' (27) verbunden sind. Die Abstände e der Faltlinien I zu der jeweils näher liegenden, parallelen Schnittkante B betragen jeweils e = 14,5 mm. In einem dritten Verfahrenschritt werden sodann die Schnittkanten A (14, 15) in Richtung der Pfeile IIa und IIb entlang der Faltlinien II (18, 19) zur Bildung von zweiten Faltkanten H (24) und H' (25) auf eine Oberseite des Materialabschnitts umgeschlagen. Die Abstände e' der Faltlinien II (18, 19) zu der jeweils näher liegenden, parallelen Schnittkante A betragen jeweils 24,5 mm. Durch das Umschlagen der Schnittkanten A (14, 15), werden die zweiten Faltkanten H (24) und H' (25), wobei jede dieser Faltkanten H oder H' zwei voneinander beabstandete Bereiche umfasst, die als Einfasskante (33a, 33b, 33a', 33b') ausgebildet sind. Des Weiteren werden aus Teilbereichen der Schnittkanten A Stoßkanten (28a, 29a; 28b, 29b; 28b', 29b') gebildet.

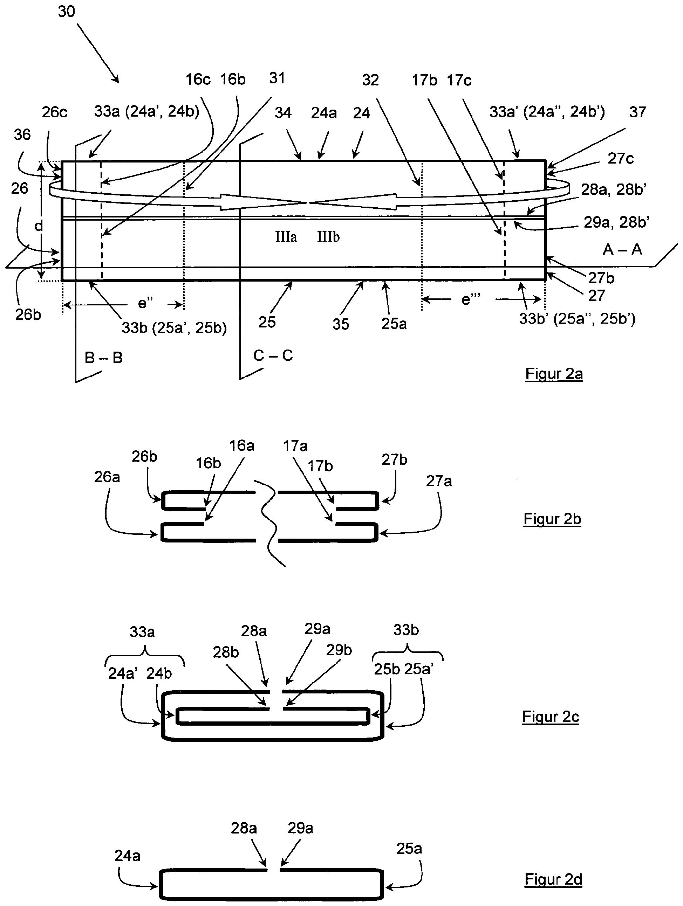

Mit Figur 2a ist das nach dem dritten Verfahrensschritt erhaltene Zwischenprodukt A (30) mit den zu den Schnittkanten B (16, 17) parallel liegenden Randkanten bb (36) und bb' (37) und den zu den Schnittkanten A (14, 15) parallel liegenden Randkanten aa (34) und aa' (35) aufgezeigt. Hierbei wird die Randkante aa (34) durch einen Teilabschnitt (24a) der zweiten Faltkante H (24) sowie durch aus weiteren Teilabschnitten (24a', 24b, 24a", 24b') der Faltkante H gebildeten Einfasskanten (33a, 33a') gebildet (vgl. auch Figur 2c - Querschnitt des Zwischenproduktes A entlang der Schnittlinie B - B sowie Figur 2d - Querschnitt des Zwischenproduktes A entlang der Schnittlinie C - C). Analog wird die Randkante aa' (35) durch einen Teilabschnitt (25a) der zweiten Faltkante H' (25) sowie durch aus weiteren Teilabschnitten (25a', 25b, 25a", 25b') der Faltkante H' gebildeten Einfasskanten (33b, 33b') gebildet. Die Randkante bb (36) wird aus übereinander liegenden Teilabschnitten (26a, 26b, 26c) der ersten Faltkante G (26) und die Randkante bb' (37) aus übereinander liegenden Teilabschnitten (27a, 27b, 27c) der ersten Faltkante G' (27) gebildet (vgl. Figur 2b - Querschnitt des Zwischenproduktes A entlang der Schnittlinie A - A). Durch das Umschlagen der Schnittkanten A (14, 15) sind aus den Schnittkanten A Stoßkanten (28a, 29a; 28b, 29b; 28b', 29b') gebildet, wobei diese Stoßkanten jeweils 1,0 mm beabstandet sind. Alle Teilbereiche der Schnittkanten B (16a, 16b, 16c, 17a, 17b, 17c) sind durch darüber liegende Materialbereiche verdeckt (in den Figuren sind durch darüber liegende Materiallagen verdeckten Kanten gestrichelt dargestellt).

In einem weiteren Verfahrensschritt wird das Zwischenprodukt A (30) weiterverarbeitet. Hierzu werden die gebildeten Randkanten bb (36) und bb' (37) des Zwischenproduktes A (30) in Richtung der Pfeile IIIa und IIIb entlang der Faltlinien III (31, 32) zur Bildung von weiteren Stoßkanten (38a, 39a, 38b, 39b) des Zwischenproduktes auf eine Oberseite des Materialabschnittes umgeschlagen. Der Abstand e" der ersten Faltlinie III (31) von der nächstliegenden, parallelen Randkante aa (36) beträgt e" = 50,0 mm. Der Abstand e'" der zweiten Faltlinie III (32) von der nächstliegenden, parallelen Randkante aa' (37) beträgt ebenfalls e'" = 50,0 mm. Damit erfolgt in diesem Verfahrensschritt ein symmetrisches Einschlagen der Randkanten aa (36) und aa' (37). Durch das Umschlagen der Randkanten bb (36) und bb' (37) werden die Einfasskanten I (41) und I' (42) sowie die Stoßkanten des Zwischenproduktes B (38a, 39a, 38b, 39b, 38c, 39c) gebildet. Hierbei ist jede gebildete Stoßkante des Zwischenproduktes (38a, 39a, 38b, 39b, 38c, 39c) aus Teilabschnitten der Faltkanten G (26) und G' (27) gebildet, so dass die Stoßkanten direkt benachbarte Lagen verbinden. Es werden keine weiteren Lagen zwischen die verbundenen Lagen ein- oder zwischengeschoben.

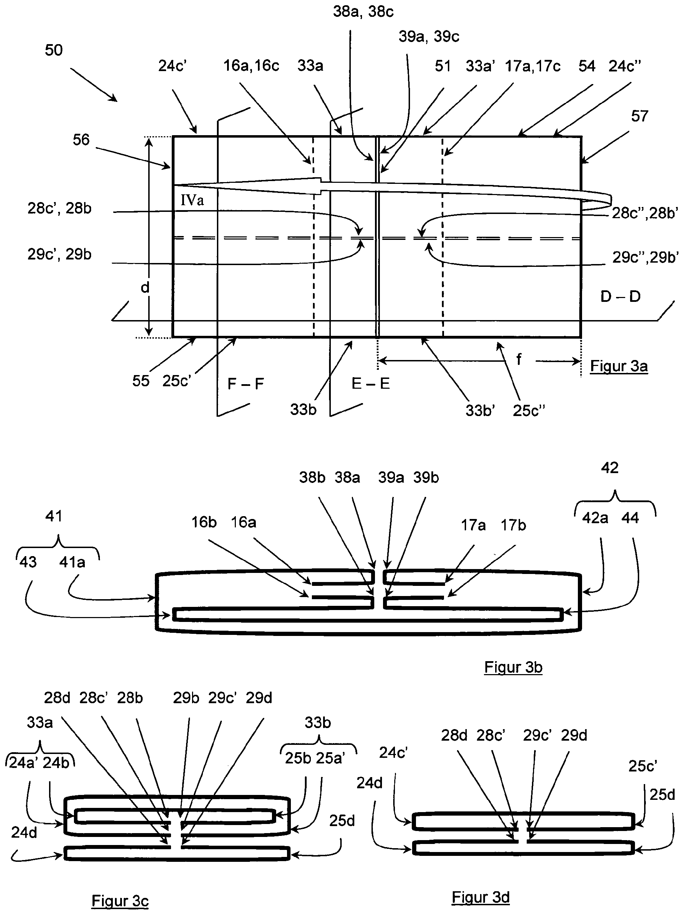

Mit Figur 3a ist das nach dem vierten Verfahrensschritt erhaltene Zwischenprodukt B (50) mit den zu den Schnittkanten B parallelen Randkanten bbb (56) und bbb' (57) sowie den zu den Schnittkanten A parallelen Randkanten aaa (54) und aaa' (55) wiedergegeben. Hierbei wird die Randkante aaa (54) durch übereinander liegende Teilabschnitte der zweiten Faltkante H (24d, 24c', 24c") sowie den im dritten Verfahrensschritt gebildeten Einfasskanten (33a, 33a') gebildet (vgl. auch Figur 3c - Querschnitt des Zwischenproduktes B entlang der Schnittlinie E - E; sowie Figur 3d - Querschnitt des Zwischenproduktes A entlang der Schnittlinie F - F). Analog wird die Randkante aaa' (55) durch übereinander liegende Teilabschnitte der zweiten Faltkante H' (25c', 25d, 25c") sowie den im dritten Verfahrensschritt gebildeten Einfasskanten (33b, 33b') gebildet. Die Randkante bbb (56) wird durch die Einfasskante I (41) und analog die Randkante aaa' (57) durch die Einfasskante I' (42) gebildet (vgl. Figur 3b - Querschnitt des Zwischenproduktes B entlang der Schnittlinie D - D). Hierbei wird die jeweils innen liegende Faltkante (43, 44) von der jeweils außen liegenden Faltkante (41a, 42a) umschlossen. Jede innen liegende Faltkante liegt direkt an der zugehörigen außen liegenden Faltkante an, so dass kein Abstand belassen ist. Das Zwischenprodukt B weist aus den Schnittkanten A bzw. aus Teilbereichen hiervon gebildete Stoßkanten (28c', 29c', 28c", 29c", 28b, 29b, 28b', 29b') auf, die parallel zu den Randkanten aaa (54) und aaa' (55) liegen. In einer weiteren Ebene des Zwischenproduktes B werden aus weiteren Teilabschnitten der Schnittkanten A weitere Stoßkanten (28d, 29d) ausgebildet. Diese Stoßkanten liegen direkt gegeneinander, wobei ein Abstand von 1,0 mm eingehalten ist. Die aus den Faltkanten G und G' gebildeten Stoßkanten (38a, 39a, 38b, 39b) weisen ebenfalls einen Abstand von 1,0 mm auf. Sowohl die Schnittkanten B bzw. jeder Teilabschnitt (16a, 16b, 16c, 17a, 17b, 17c) als auch jede aus einem Teilabschnitt der Schnittkanten A gebildete Stoßkante (28b, 29b, 28c', 29c', 28c", 29c", 28d, 29d, 28b', 29b') werden durch darüber liegende Materiallagen verdeckt. Somit liegen in diesem Zwischenprodukt B (50) keine freien Schnittkanten vor.

In einem letzten Verfahrensschritt wird das Zwischenprodukt B (50) weiterverarbeitet. Hierbei wird die zuvor durch die Einfasskante I' (42) gebildete Randkante bbb' (57) des Zwischenproduktes B (50) in Richtung des Pfeils IVa entlang der Faltlinie IV (51) umgeschlagen, so dass die umgeschlagene Randkante bbb' (57) auf die durch die Einfasskante I gebildeten Randkante bbb (56) des Zwischenproduktes B (50) aufgelegt wird. Der Abstand der Faltlinie IV (51) zu der Randkante bbb (56) entspricht f = 50,5 mm. Damit liegt die Faltlinie IV (51) exakt zwischen einem Stoßkantenpaar (38a, 39a; 38b, 39b; 38c, 38c). Durch diesen Verfahrensschritt werden entlang der Faltlinie IV (51) die weiteren Faltkanten J (47a) und J' (46a) gebildet. Die Stoßkanten (38a, 39a; 38b, 39b; 38c, 39c) des Zwischenproduktes B (50) bildenden Faltkanten G und G' bzw. deren Teilbereiche (26a, 26b, 27a, 27b, 26c, 27c) sind nur gegen die Faltkante J' (46a) angelegt. Zwischen jedem der Teilbereiche der Faltkanten G oder G' und der Faltkante J' (46a) ist kein Abstand gelassen, so dass die beteiligten Kanten direkt aneinander liegen. Damit wird in diesem Verfahrensschritt auch die Einfasskante K' (46) gebildet.

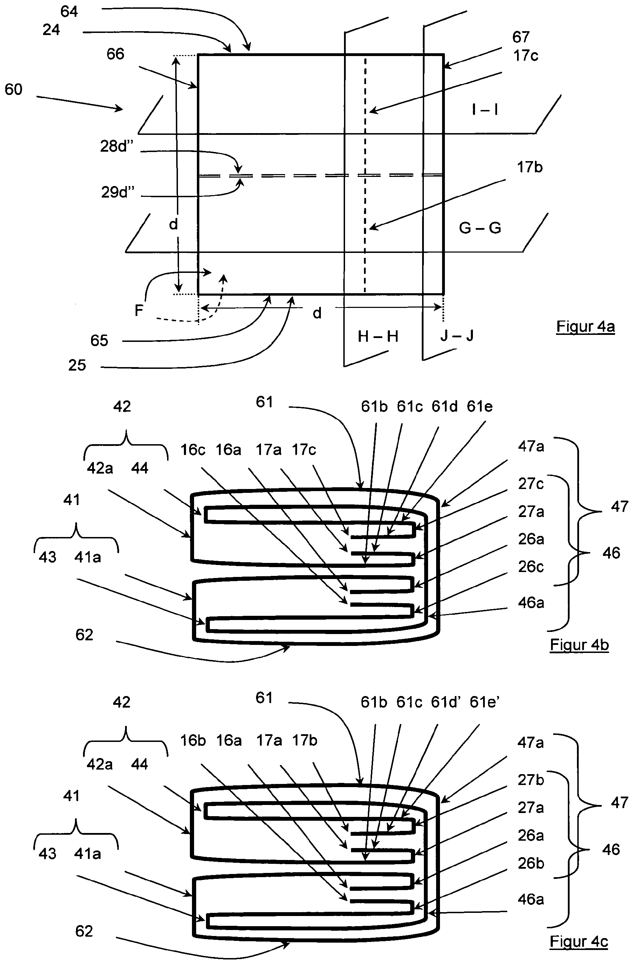

Mit Figur 4a ist die im zuvor beschriebenen Verfahren hergestellte Kompresse (60) abgebildet. Die Kompresse mit den vier gleichlangen Randkanten D', D", D'" und D"" (64, 65, 66 und 67) mit einer Kantenlänge d = 50,0 mm weist quadratische Auflageflächen F mit dem Flächenmaß d² = 25,0 cm² auf. Alle Teilbereiche der Schnittkanten B und alle Teilbereiche der Schnittkanten A sind durch weitere Materiallagen verdeckt. In Figur 4a sind nur die oben liegenden Teilbereiche der Schnittkante B (17b, 17c) sowie oben liegenden Teilbereiche der als Stoßkanten ausgebildeten Schnittkanten A (28d", 29d") wiedergegeben. Die aus einem Teilbereich der Faltkante H (24) und Teilbereich der Faltkante H' (25) gebildeten Stoßkanten (28d", 29d") liegen direkt aneinander, wobei diese einen Abstand von 1,0 mm aufweisen. Dies entspricht einem Abstand von 2 % bezogen auf irgendeine Randkante der endgefertigten Kompresse (alle Kanten sind gleich lang). Die zweiten Schnittkanten B bzw. Teilabschnitte hiervon (16a, 16b, 16c, 17a, 17b, 17c) liegen deckungsgleich übereinander, wobei der Abstand zur ersten parallel liegenden Randkante D' (67) in jedem Punkt 14,5 mm beträgt. Dies entspricht 29 % des Betrags der Länge einer zweiten, senkrecht zur ersten Randkante liegenden zweiten Randkante D" (64). auf.

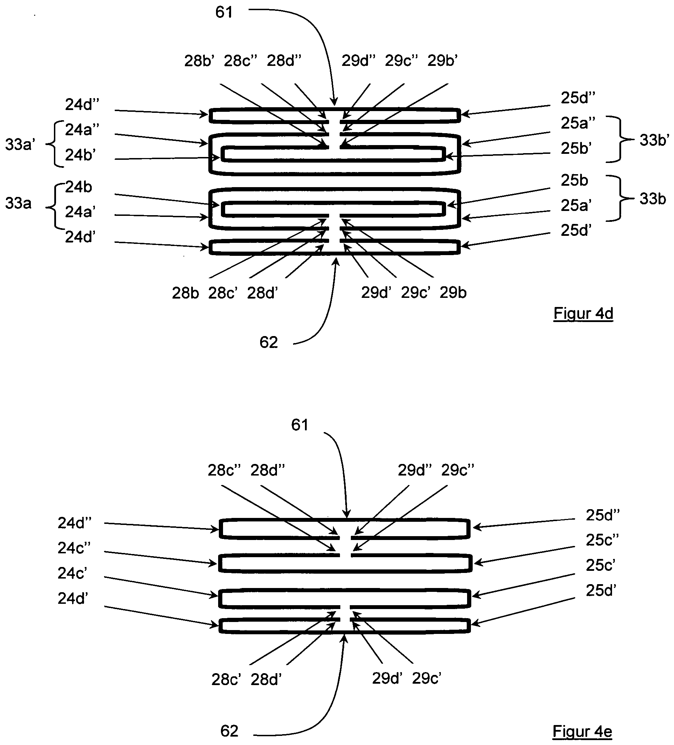

Mit den folgenden Figuren 4b, 4c, 4d und 4e soll die Lagigkeit der Kompresse (60) sowie die zuletzt gebildeten Einfasskanten verdeutlicht werden, wobei mit Figur 4b der Querschnitt gemäß Schnittlinie I - I, mit Figur 4c der Querschnitt gemäß Schnittlinie G - G, mit Figur 4d der Querschnitt gemäß Schnittlinie J - J und mit Figur 4e der Querschnitt gemäß Schnittlinie H - H gezeigt ist. Zur Verdeutlichung sind die einzelnen Lagen sowie ineinander liegende Faltkanten - wie in allen Querschnittszeichnungen - auseinander gezogen bzw. voneinander beabstandet dargestellt. Insbesondere ist gezeigt, dass die beiden äußeren die Auflageflächen F bildenden Lagen (61, 62) durch die Faltkante J (47a) miteinander verbunden sind. Diese Lagen werden jeweils durch zusammenhängende Materialbereiche des Materialabschnitts gebildet. Jede weitere Lage ist von diesen beiden äußeren Lagen ummantelt, so dass alle weiteren Lagen zwischen den äußeren Lagen (61, 62) liegen.

Des Weiteren ist die Einfasskante K (47), die die erste Randkante D' der Kompresse bildet gezeigt. Die Einfasskante K (47) ist aus den innen liegenden Faltkanten G und G' bzw. deren Teilabschnitten (26a, 26b, 27a, 27b, 26c, 27c) und der außen liegenden Faltkante J (47a) gebildet, wobei die innen liegenden Faltkanten von der außen liegenden Faltkante durch die weitere Faltkante J' (46a), die ebenfalls mit den innen liegenden Faltkanten eine Einfasskante K' (46) bildet, beabstandet sind. Da der Abstand der Dicke des eingesetzten Materials entspricht, die kleiner als 0,5 mm ist, ist der Abstand vernachlässigbar, so dass die innen liegenden Kanten auch direkt an der außen liegenden Faltkante anliegen. Jede der innen liegenden Faltkanten G oder G' bzw. deren Teilabschnitte (26a, 26b, 27a, 27b, 26c, 27c) verbindet zwei direkt benachbarte Lagen bzw. Teilabschnitte der Lagen (vgl. Figur 4b und 4c). So verbinden beispielsweise die ersten Teilabschnitte (27b, 27c) der Faltkante G' (27) jeweils einen Teilabschnitt der dritten Lage (61e, 61e') mit jeweils einem Teilabschnitt der vierten Lage (61d, 61d') der Kompresse. Ein weitere Teilabschnitt (27a) verbindet die fünfte Lage (61c) mit der sechsten Lage (61b) der Kompresse. Senkrecht zu der Einfasskante K (47) oder K' (46) liegen die aus den Schnittkanten A gebildeten Stoßkanten (28d", 29d") und weitere aus den Schnittkanten A bzw. deren Teilabschnitte gebildete Stoßkanten (28c', 29c'; 28c", 29c"; 28d', 29d'; 28b, 29b; 28b', 29b').

Des Weiteren ist gezeigt, dass die zweite Randkante D" (64) der Kompresse aus übereinander liegenden Teilabschnitten der Faltkante H (24c', 24d', 24c", 24d") und den im dritten Verfahrensschritt gebildeten Einfasskanten (33a, 33a'), die dritte Randkante D'" (45) aus übereinander liegenden Teilabschnitten der Faltkante H' (25c', 25d', 25c", 25d") und den im dritten Verfahrensschritt gebildeten Einfasskanten (33b, 33b')und die vierte Randkante D"" (66) aus den übereinander liegenden Einfasskanten I (41) und I' (42) gebildet ist. Alle übereinander liegenden Kanten liegen in Aufsicht betrachtet deckungsgleich. Damit weist die Kompresse lediglich solche Randkanten auf, die durch Faltkanten oder Einfasskanten gebildet werden. Insbesondere bildet keine Schnittkanten des Materialabschnitts (10) eine Randkante der Kompresse (60). Jede Lage dieser Kompresse ist mittels mindestens einer Faltkante mit einer weiteren Lage der Kompresse verbunden, wobei die Kompresse im Querschnitt betrachtet einen ersten und einen zweiten Randbereich aufweist. Der erste Randbereich ist 8-lagig ausgebildet, wogegen der zweite Randbereich 12-lagig ausgebildet ist. Die vier zusätzlichen Lagen in dem zweiten Randbereich werden durch das Umschlagen von den Schnittkanten B (16, 17) erhalten (vgl. Figur 4b, 4c).

Diese Kompresse weist als 8-lagige Kompresse auch nach einmaligem Entfalten keine freien Schnittkanten auf (vgl. Figur 3a). Somit könnte diese Kompresse als 4-lagige oder 8-lagige Kompresse verwendet werden. Die Kompresse (60) weist im ersten Randbereich (8 Lagen) eine Dicke vom 1,12 mm auf und im zweiten Randbereich, (12 Lagen) eine Dicke von 1,36 mm, jeweils gemessen bei einem Prüfdruck von 2 g/cm² (vgl. unten). Damit kann eine Mehrzahl dieser Kompressen leicht gestapelt werden, da sie über alle Bereiche eine gleichmäßige Materialverteilung aufweist.

Im Folgenden soll ein Vergleich der Stapelhöhen angegeben werden. Werden beispielsweise im Markt befindliche ES-Kompressen (ES-Kompressen 5x5 cm - Paul Hartmann AG) gestapelt, so weist die Verpackung mit einem Kompressenstapel von 100 Kompressen eine Höhe von 155 mm (Außenmaß der Verpackung) auf. Werden dagegen 100 Stück erfindungsgemäße Kompressen (60) gestapelt und unter denselben Bedingungen mit demselben Verpackungsmaterial verpackt, so ist ein Außenmaß von 139 mm zu verzeichnen (dieselben Messbedingungen). Das entspricht einer Reduzierung in der Stapelhöhe von ca. 10 %.

Für einen Teilstapel von 5 Kompressen bzw. jede einzelne Kompresse ergeben sich folgende Werte, wobei ein Prüfdruck 2g/ cm² eingehalten wurde. Jede Kompresse ist identisch gefaltet, wobei die Kompressen im Stapel deckungsgleich gestapelt sind.

| | | Dicke (5 Stück) / mm | Dicke (1 Stück) / mm |
|---|---|---|---|
| ES-Kompresse (Paul Hartmann AG) | Randkante (16 Lagen) | 8,10 | 1,62 |
| | parallele Gegenkante (8 Lagen) | 5,65 | 1,13 |
| Erfindungsgemäße Kompresse (60) | erster Randbereich (8 Lagen) | 5,60 | 1,12 |
| | zweiter Randbereich (12 Lagen) | 6,81 | 1,36 |

Durch die Anordnung der Faltkanten kann ein Kompressenstapel bereitgestellt werden, der im Vergleich zu im Markt befindlichen Kompressen in sich stabiler ist und weniger Platz einnimmt.

In der folgenden Tabelle sind Beispiele quadratischer Kompressen angegeben, die gemäß des oben beschriebenen Verfahrens aus einem rechteckigen Materialabschnitt mit zwei sich gegenüber liegenden parallelen Schnittkanten A und zwei sich gegenüber liegenden parallelen Schnittkanten B gebildet sind. Die Kantenlänge a der Schnittkanten A ist größer als die Kantenlänge b der Schnittkanten B des bereitgestellten Materialabschnitts. Die verglichenen Kompressen weisen im endgefalteten Zustand die jeweils angegebene Randkantenlänge d auf. Anhand der Tabelle soll verdeutlicht werden, inwieweit bei gleichem Material eine Materialersparnis gegenüber bekannten ES-Kompressen - Paul Hartmann AG (1. Faltung) verwirklicht ist.

| Fläche des eingesetzten Materialabschnitts F_{M} = a · b | Kompresse 1 d = 5 cm | | | Kompresse 2 d = 7,5 cm | Kompresse 3 d = 10 cm |
|---|---|---|---|---|---|
| | e = 1,0 cm | e = 1,5 cm | e = 2,0 cm | e = 1,5 cm | e = 1,5 cm |
| 1. Faltung ES-Kompresse F_{M} = 4d · (2d + 2e) | 240 cm² | 260 cm² | 280 cm² | 540 cm² | 920 cm² |
| 2. Faltung (erfindungsgemäß) F_{M} = (4d + 2e) · 2d | 220 cm² | 230 cm² | 240 cm² | 495 cm² | 860 cm² |
| Materialersparnis | 20 cm² (9,1%) | 30 cm² (11,5%) | 40 cm² (14,3%) | 45 cm² (8,3%) | 60 cm² (6,5%) |

So beläuft sich beispielsweise die Materialersparnis für eine erfindungsgemäße Kompresse mit quadratischen Auflageflächen und einer Kantenlänge d mit d = 5 cm (Kompresse 1) bei gleicher Breite e des im ersten Schritt umgeschlagenen Abschnittes mit e = 1,5 cm auf ca. 11,5% gegenüber etablierten ES-Kompressen. Sowohl die Stoßkanten als auch die Einfasskanten der erfindungsgemäßen Kompressen weisen keine Abstände auf. Die Materialersparnis kann somit durch präzise Faltung in neuartiger Art erzielt werden, wobei durch die Faltung eine erfindungsgemäße Einfasskante in der Kompresse verwirklicht ist.

Diese hier gezeigten medizinischen Kompressen können insbesondere in der Notfallmedizin als auch bei operativen Eingriffen verwendet werden. Sie zeichnen sich durch eine besondere Sicherheit im Gebrauch und durch eine besonders gleichmäßige Materialverteilung aus.

## Patentansprüche

1. Medizinische Kompresse (60) umfassend mindestens 8 Lagen eines textilen Flachbahnmaterials, wobei jede Lage über mindestens eine Faltkante mit einer weiteren Lage verbunden ist, und mindestens zwei Faltkanten senkrecht zueinander stehen, und die Kompresse aus einem rechteckigen Materialabschnitt des Flachbahnmaterials (10) mit zwei sich gegenüber liegenden parallelen Schnittkanten A (14, 15) und zwei sich gegenüber liegenden parallelen Schnittkanten B (16, 17) gebildet ist, wobei die Kantenlänge a der Schnittkanten A größer ist als die Kantenlänge b der Schnittkanten B, **dadurch gekennzeichnet, dass** die Kompresse derart gefaltet ist, dass die Kompresse eine aus mindestens einer ersten Faltkante (26a, 26b, 26c, 27a, 27b, 27c) und einer zweiten Faltkante (47a) gebildete erste Einfasskante (47) umfasst, wobei die erste Faltkante (26a, 26b, 26c, 27a, 27b, 27c) direkt benachbarte Lagen oder Teilabschnitte der direkt benachbarten Lagen verbindet und parallel zu den Schnittkanten B (16, 17) gebildet ist, und die zweite Faltkante die äußeren, die Auflageflächen (61, 62) der Kompresse bildenden Lagen verbindet und wobei jede Randkante (64, 65, 66, 67) der Kompresse ausschließlich aus Faltkanten oder Einfasskanten gebildet ist, wobei die Kompresse im Querschnitt betrachtet einen ersten 8-lagigen Randbereich und einen zweiten 12-lagigen Randbereich aufweist und die Kompresse derart gefaltet ist, dass die äußeren, die Auflageflächen der Kompresse bildenden Lagen die Form eines Rechtecks oder eines Quadrates aufweisen.

2. Medizinische Kompresse nach Anspruch 1, **dadurch gekennzeichnet, dass** die erste Einfasskante (47) mindestens zwei, insbesondere vier direkt benachbarte Lagen verbindende erste Faltkanten (26a, 26b, 26c, 27a, 27b, 27c) umfasst.

3. Medizinische Kompresse nach mindestens einem der vorgenannten Ansprüche, **dadurch gekennzeichnet, dass** die Kompresse derart gefaltet ist, dass die äußeren, die Auflageflächen der Kompresse bildenden Lagen (61, 62) vollständig durch jeweils einen zusammenhängenden Bereich des Flachbahnmaterials gebildet sind.

4. Medizinische Kompresse nach mindestens einem der vorgenannten Ansprüche, **dadurch gekennzeichnet, dass** Teilabschnitte der Schnittkanten B (16a, 16b, 16c, 17a, 17b, 17c) in Aufsicht der Kompresse betrachtet innerhalb der Kompresse deckungsgleich angeordnet liegen.

5. Medizinische Kompresse nach mindestens einem der vorgenannten Ansprüche, **dadurch gekennzeichnet, dass** die Schnittkanten B oder ein Teilabschnitt der Schnittkanten B (16, 17, 16a, 16b, 16c, 17a, 17b, 17c) in jedem Punkt einen Abstand zu der Einfasskante (47) von mindestens 20 % und höchstens 80 % des Betrags der Länge einer senkrecht zur der Einfasskante liegenden Randkante (64, 65) aufweist.

6. Medizinische Kompresse nach mindestens einem der vorgenannten Ansprüche, **dadurch gekennzeichnet, dass** die Kompresse zwei Schnitt- oder Faltkanten als Stoßkanten (28d", 29d") umfasst.

7. Medizinische Kompresse nach mindestens einem der vorgenannten Ansprüche, **dadurch gekennzeichnet, dass** die Kompresse derart gefaltet ist, dass die Schnittkanten A oder Teilbereiche der Schnittkanten A als Stoßkanten (28d", 29d") ausgebildet sind.

8. Kompressenstapel umfassend eine Mehrzahl von Kompressen (60) gemäß mindestens einem der vorgenannten Ansprüche.

## Claims

1. Medical compress (60) comprising at least 8 layers of a textile flat web material, wherein each layer is connected to a further layer via at least one folded edge, and at least two folded edges are perpendicular to each other, and the compress is formed from a rectangular material section of the flat web material (10) having two parallel cut edges A (14, 15) lying opposite each other and having two parallel cut edges B (16, 17) lying opposite each other, wherein the edge length a of the cut edges A is greater than the edge length b of the cut edges B, **characterized in that** the compress is folded in such a way that the compress comprises a first enclosing edge (47) formed from at least one first folded edge (26a, 26b, 26c, 27a, 27b, 27c) and one second folded edge (47a), wherein the first folded edge (26a, 26b, 26c, 27a, 27b, 27c) connects directly adjacent layers or partial sections of the directly adjacent layers and is formed parallel to the cut edges B (16, 17), and the second folded edge connects the outer layers forming the contact surfaces (61, 62) of the compress, and wherein each outer edge (64, 65, 66, 67) of the compress is formed exclusively from folded edges or enclosing edges, wherein the compress, viewed in cross section, has a first 8-layer edge region and a second 12-layer edge region, and the compress is folded in such a way that the outer layers forming the contact surfaces of the compress have the shape of a rectangle or of a square.

2. Medical compress according to Claim 1, **characterized in that** the first enclosing edge (47) comprises at least two, in particular four, first folded edges (26a, 26b, 26c, 27a, 27b, 27c) connecting directly adjacent layers.

3. Medical compress according to at least one of the preceding claims, **characterized in that** the compress is folded in such a way that the outer layers (61, 62) forming the contact surfaces of the compress are formed completely by in each case one contiguous region of the flat web material.

4. Medical compress according to at least one of the preceding claims, **characterized in that** partial sections of the cut edges B (16a, 16b, 16c, 17a, 17b, 17c), viewed in a plan view of the compress, lie congruently within the compress.

5. Medical compress according to at least one of the preceding claims, **characterized in that** the cut edges B or a partial section of the cut edges B (16, 17, 16a, 16b, 16c, 17a, 17b, 17c) have, at each point, a distance from the enclosing edge (47) of at least 20% and at most 80% of the length of an outer edge (64, 65) lying perpendicular to the enclosing edge.

6. Medical compress according to at least one of the preceding claims, **characterized in that** the compress comprises two cut or folded edges as abutment edges (28d", 29d").

7. Medical compress according to at least one of the preceding claims, **characterized in that** the compress is folded in such a way that the cut edges A or partial regions of the cut edges A are configured as abutment edges (28d", 29d").

8. Stack of compresses, comprising a plurality of compresses (60) according to at least one of the preceding claims.

## Revendications

1. Compresse médicale (60) comprenant au moins 8 couches d'un matériau textile en bande plate, chaque couche étant reliée via au moins un bord plié à une autre couche et au moins deux bords pliés étant perpendiculaires l'un à l'autre et la compresse étant formée par un morceau rectangulaire du matériau en bande plate (10) présentant deux bords de coupe A (14, 15) parallèles l'un en face de l'autre et deux bords de coupe B (16, 17) parallèles l'un en face de l'autre, la longueur de bord a des bords de coupe A étant supérieure à la longueur de bord b des bords de coupe B, **caractérisée en ce que** la compresse est pliée de manière telle que la compresse comprend un premier bord d'encadrement (47) formé par au moins un premier bord plié (26a, 26b, 26c, 27a, 27b, 27c) et un deuxième bord plié (47a), le premier bord plié (26a, 26b, 26c, 27a, 27b, 27c) reliant des couches directement adjacentes ou des sections partielles directement adjacentes des couches directement adjacentes et étant formé parallèlement aux bords de coupe B (16, 17) et le deuxième bord plié reliant les couches externes, formant les surfaces support (61, 62) de la compresse et chaque bord périphérique (64, 65, 66, 67) de la compresse étant formé exclusivement par des bords pliés ou des bords d'encadrement, la compresse présentant, vue en coupe transversale, une première zone périphérique de 8 couches et une deuxième zone périphérique de 12 couches et la compresse étant pliée de manière telle que les couches externes, formant les surfaces support de la compresse, présentent la forme d'un rectangle ou d'un carré.

2. Compresse médicale selon la revendication 1, **caractérisée en ce que** le premier bord d'encadrement (47) comprend au moins deux, en particulier quatre, premiers bords pliés (26a, 26b, 26c, 27a, 27b, 27c) reliant des couches directement adjacentes.

3. Compresse médicale selon au moins l'une quelconque des revendications susmentionnées, **caractérisée en ce que** la compresse est pliée de manière telle que les couches (61, 62) externes, formant les surfaces support de la compresse, sont formées complètement par à chaque fois une zone cohérente du matériau en bande plate.

4. Compresse médicale selon au moins l'une quelconque des revendications susmentionnées, **caractérisée en ce que** les sections partielles des bords de coupe B (16a, 16b, 16c, 17a, 17b, 17c), vues à partir du dessus de la compresse, sont agencées en superposition dans la compresse.

5. Compresse médicale selon au moins l'une quelconque des revendications susmentionnées, **caractérisée en ce que** les bords de coupe B ou une section partielle des bords de coupe B (16, 17, 16a, 16b, 16c, 17a, 17b, 17c) présente(nt) en chaque point une distance par rapport au bord d'encadrement (47) d'au moins 20% et d'au plus 80% de la valeur de la longueur d'un bord périphérique (64, 65) perpendiculaire au bord d'encadrement.

6. Compresse médicale selon au moins l'une quelconque des revendications susmentionnées, **caractérisée en ce que** la compresse comprend deux bords de coupe ou bords pliés en tant qu'arêtes de contact (28d", 29d").

7. Compresse médicale selon au moins l'une quelconque des revendications susmentionnées, **caractérisée en ce que** la compresse est pliée de manière telle que les bords de coupe A ou des zones partielles des bords de coupe A sont conçu(e)s en tant qu'arêtes de contact (28d", 29d").

8. Pile de compresses comprenant une multitude de compresses (60) selon au moins l'une quelconque des revendications susmentionnées.
